# EUROPÄISCHE PATENTANMELDUNG

(11) **EP 2 617 356 A1**
(43) Veröffentlichungstag der Anmeldung: **24.07.2013**
(21) Anmeldenummer: 12151541.5
(22) Anmeldetag: 18.01.2012
(51) Int. Cl.: A61B 5/1455, A61B 5/145

(54) **Analytisches System zur Untersuchung einer Körperflüssigkeit und Verfahren zu dessen Betrieb**

(71) Anmelder: Roche Diagnostics GmbH, 68305 Mannheim (DE); F. Hoffmann-La Roche AG, 4070 Basel (CH)
(72) Erfinder: List, Hans, 64754 Hesseneck-Kailbach (DE)
(74) Vertreter: Pfiz, Thomas

(57) **Zusammenfassung**

Die Erfindung betrifft ein analytisches System sowie ein Verfahren zur Untersuchung einer Körperflüssigkeit mit einem als Verbrauchsmittel wechselbaren Magazin (18), das eine Mehrzahl von mit jeweils mindestens einem analytischen Hilfsmittel (32,34) und einem Transportelement (28) versehene Magazineinheiten (24) umfasst, einem handgehaltenen Gerät (12) mit einer Magazinführung (16), und einem an den Transportelementen (28) angreifenden Transportmechanismus (48) zum schrittweisen Transport des Magazins (18) in der Magazinführung (16). Erfindungsgemäß ist eine über Haltemittel (52,54) mit Transportelementen (28) des Magazins (18) in Eingriff bringbare Positioniereinrichtung (50) zur Positionierung einer aktiven Magazineinheit (24) in einer vorgegebenen Funktionsposition vorgesehen.

## Beschreibung

Die Erfindung betrifft ein analytisches System zur Untersuchung einer Körperflüssigkeit, insbesondere für Blutzuckertests, mit einem als Verbrauchsmittel wechselbaren Magazin, das eine Mehrzahl von mit jeweils mindestens einem analytischen Hilfsmittel und einem Transportelement versehene Magazineinheiten umfasst, einem handgehaltenen Gerät mit einer Magazinführung zur Aufnahme des Magazins, und einem an den vorzugsweise ringförmig angeordneten Transportelementen angreifenden Transportmechanismus zum schrittweisen Transport des Magazins in der Magazinführung. Die Erfindung betrifft weiter ein Verfahren zum Betrieb eines solchen analytischen Systems.

In tragbaren handgehaltenen Systemen zur automatischen Gewinnung und Messung von Körperflüssigkeiten werden zunehmend Magazine mit mehreren disposiblen Messeinheiten eingesetzt, um die Benutzungsfreundlichkeit zu verbessern. Dabei ergibt sich gegenüber herkömmlichen Messgeräten ein erhöhter Aufwand an mechanischen Bewegungen. In dem Bestreben, die Baugröße solcher Systeme zu minimieren, werden die Strukturen des Magazins und des Gerätes, die zueinander passen müssen, immer kleiner, wenngleich die Toleranzen der Einzelteile nicht im gleichen Maßstab schwinden. Damit werden die Anforderungen an die Positionierung immer schärfer. Die einzelnen miniaturisierten Tests fordern sowohl bezüglich der Relativposition zum Auswertesystem eine hohe Präzision und Richtigkeit als auch bezüglich der Stabilität der Position während der Messung eine große Robustheit. Letzteres gilt bei elektrisch ausgewerteten Tests beinahe noch stärker, weil jede Bewegung von stromdurchflossenen Kontakten zu Signalrauschen führt, was die Auswertung stört oder völlig unmöglich macht. Aber auch optische Systeme sind höchst empfindlich gegen Positionsveränderungen während der laufenden Messung. Als weitere Randbedingung ist zu beachten, dass das Magazin als Verbrauchsmittel auf die für den Testablauf unumgänglichen Strukturen und Bauteile beschränkt bleiben sollte, damit der Herstellungsaufwand so niedrig wie möglich gehalten werden kann.

Ein gattungsgemäßes analytisches System mit einem Magazin in der Form einer Kreisringscheibe ist in der WO 2010/094426 erwähnt. Dort wird vorgeschlagen, dass das analytische System über eine entsprechende Transportvorrichtung verfügen kann, welche mit Transportelementen des Magazins zusammenwirkt, um einen Weitertransport der Magazineinheiten zu bewirken. In diesem Dokument ist auch ein Verfahren zur Herstellung eines solchen Magazins offenbart, auf das hier Bezug genommen wird.

Allgemein führen Konzepte, ein ringförmiges Magazin über das Ringzentrum im Gerät aufzunehmen und die Präzision der Bewegungen den Geräteteilen zu überlassen, zu einer sehr aufwendigen und teuren Bauform, da neben den unvermeidlichen Maßabweichungen der Magazinscheibe selbst noch eine Fülle von Strukturen des Gerätes, die ebenfalls notwendig Maßabweichungen und überdies teilweise Laufspiel aufweisen, in die Toleranzkette der Positionierung mit aufgenommen werden. Die sechs Bewegungsfreiheitsgrade des Magazins können so nur mit kaum vertretbarem Aufwand unter Kontrolle gebracht werden.

Ausgehend hiervon liegt der Erfindung die Aufgabe zugrunde, die im Stand der Technik vorgeschlagenen Systeme und Verfahren weiter zu verbessern und eine genaue Positionierung der Test- bzw. Magazineinheiten zu gewährleisten, so dass auch bei starker Miniaturisierung die mechanischen und messtechnischen Anforderungen erfüllt werden. Zugleich soll sowohl das Auswechseln als auch der Transport des Magazins mit einfachen Mitteln möglich sein.

Zur Lösung dieser Aufgabe wird die im Patentanspruch 1 bzw. 15 angegebene Merkmalskombination vorgeschlagen.

Vorteilhafte Ausgestaltungen und Weiterbildungen der Erfindung ergeben sich aus den abhängigen Ansprüchen.

Die Erfindung geht von dem Gedanken aus, einer exakten Positionierung während der Messung oberste Priorität einzuräumen. Dementsprechend wird erfindungsgemäß eine über Haltemittel mit Transportelementen des Magazins in Eingriff bringbare Positioniereinrichtung des Geräts zur Positionierung einer zur Benutzung bereitgestellten aktiven Magazineinheit in einer vorgegebenen gerätefesten Funktionsposition vorgeschlagen. Die Haltemittel und die Transportelemente lassen sich somit in eine gegenseitige Eingriffstellung bringen, in der ein Magazintransport unterbrochen und zugleich eine genaue gerätebezogene Positionshaltung erreicht wird, so dass insbesondere eine Relativposition zu einer Messeinheit oder einer Fingeraufnahme oder dergleichen mit geringen Toleranzen reproduzierbar eingehalten werden kann.

Vorteilhafterweise greifen Haltemittel an dem Transportelement der in der Funktionsposition befindlichen aktiven Magazineinheit formschlüssig an und verhindern damit eine Bewegung des Magazins in mindestens einer Richtung. Dadurch ist es möglich, geringe Abstände zu relevanten Bezugspunkten einzuhalten und damit auch die Variationen solcher Abstände zu minimieren. Hierbei ist auch zu berücksichtigen, dass speziell in Massenherstellverfahren wie Spritzguss von Verbrauchsmitteln die Abweichungen mit der Größe eines Maßes korrelieren.

Vorteilhafterweise sind die Transportelemente ringförmig angeordnet, während die Haltemittel in der Eingriffstellung mit zwei einander vorzugsweise diametral gegenüberliegenden, zumindest aber voneinander weit entfernt angeordneten Transportelementen in Formschlussverbindung stehen und somit eine Bewegung in mehreren Freiheitsgraden verhindern. Denkbar ist es auch, dass die durch Haltemittel in Eingriff genommenen Transportelemente nicht auf einer Achse, sondern unter einem stumpfen Winkel bezüglich des Magazinzentrums liegen.

Besonders bevorzugt sind zwei Haltemittel vorgesehen, wobei bevorzugt ein erstes Haltemittel gerätefest und ein zweites Haltemittel in dem Gerät beweglich angeordnet ist.

Eine besonders vorteilhafte Ausgestaltung sieht eine in dem Gerät starr angeordnete Lagerstelle als Haltemittel zur Aufnahme des Transportelements der aktiven Magazineinheit vor. Damit wird ein fester Bezugspunkt im Gerät geschaffen, so dass weitere Geräteeinrichtungen relativ dazu ausgerichtet werden können. Dies lässt sich auch hinsichtlich des anschließenden Transportvorgangs vorteilhaft dadurch realisieren, dass die Haltemittel ein seitlich offenes Lagerauge aufweisen, in welches das Transportelement der in der Funktionsposition befindlichen Magazineinheit seitlich in einer Linearbewegung einrückbar und wieder ausrückbar ist.

Vorteilhafterweise ist das ringförmige Magazin in der Lagerstelle um eine durch das Transportelement der aktiven Magazineinheit verlaufende exzentrische Achse begrenzt schwenkbeweglich gelagert, so dass auch für die Weiterschaltung der Magazineinheiten während einer Transportphase eine definierte Gerätestruktur genutzt werden kann und das Magazin nicht sogleich vollständig freibeweglich wird.

Um einen gezielt schaltbaren Eingriff zu ermöglichen, ist es von Vorteil, wenn die Haltemittel einen zwischen einer mit einem Transportelement in Eingriff befindlichen Eingriffsstellung und einer davon entfernten Freigabestellung beweglichen Greifer zur Lagesicherung des Magazins umfassen.

Dabei sollte zur weiteren Reduzierung eines Positionierspiels der Greifer in seiner Eingriffsstellung unter Ausübung einer Kraft gegen mindestens ein Transportelement andrücken. Grundsätzlich ist es auch möglich, dass der Greifer in seiner Eingriffsstellung unter Ausübung einer Kraft zwischen zwei Transportelemente greift und gegen diese andrückt.

Günstig ist es auch, wenn der Transportmechanismus ein Steuermittel, insbesondere ein Kurvengetriebe zur Bewegungssteuerung des Greifers aufweist. Damit lassen sich gegebenenfalls mit nur einer Antriebsquelle, beispielsweise einem Elektromotor sowohl Transport- als auch Positionierabläufe gemeinsam steuern und aufeinander abstimmen.

Um einen Magazinwechsel zu erleichtern, ist es vorteilhaft, wenn der Greifer über ein Stellmittel mit einem die Magazinführung verschließenden Deckel gekoppelt ist, so dass der Greifer beim Öffnen des Deckels in die Freigabestellung gelangt, wodurch insgesamt fünf der sechs Freiheitsgrade des Magazins so weit freigegeben werden und der Anwender das Magazin problemlos auswechseln kann.

Zur Vorpositionierung ist es besonders vorteilhaft, wenn die Magazinführung drei eine Ebene aufspannende Auflagepartien zur Auflagerung einer ebenen Führungsfläche des Magazins aufweist.

Eine weitere Verbesserung der Lagegenauigkeit kann dadurch erreicht werden, dass das in einer Lagerebene begrenzt bewegbare Magazin unter der Einwirkung einer Normalkraft zu der Lagerebene steht. Vorteilhaft ist es, wenn die lotrechten Kräfte auf die Arbeitsebene des Magazins durch Federn im Deckel, der den Arbeitsraum des Magazins verschließt, erzeugt werden. Des Weiteren ist es günstig, wenn die lotrechten Kräfte in Summe so groß sind wie das Produkt aus der Masse des Magazins und der maximal zulässigen Stoßbeschleunigung, die eine Messung noch nicht beeinträchtigen soll.

Vorteilhafterweise ist der Transportmechanismus mit Getriebemitteln versehen, die einer exzentrischen Schwenkbewegung des Magazins eine begrenzte lineare Ausrückbewegung überlagern, wobei mindestens ein Transportelement außer Eingriff mit einem gerätefesten Haltemittel gelangt. Damit kann insbesondere ein Ringmagazin abwechselnd schrittweise transportiert und positioniert werden.

Im Zusammenhang mit einer kombinierten Dreh- und Linearbewegung ist es auch vorteilhaft, wenn der Transportmechanismus einen auf einer elliptischen Bahn bewegbaren und in einem Abschnitt seiner Bewegungsbahn an einem Transportelement angreifenden Schieber aufweist, und wenn die Magazinführung bogenförmige Leitkonturen für die Transportelemente aufweist, wobei die Leitkonturen eine von einer Kreisbahn abweichende Ausrückbewegung der Transportelemente steuern.

Zur Gebrauchsprüfung der Magazineinheiten ist eine Prüfeinheit insbesondere in Form einer eine Siegelfolie abtastenden Lichtschranke von Vorteil.

Bevorzugt ist das Magazin als Ringmagazin, insbesondere als kreisringförmiges Scheibenmagazin mit in Umfangsrichtung verteilten Magazineinheiten ausgebildet. Ein solches Magazin hat vorteilhafterweise einen Durchmesser von weniger als 100 mm, bevorzugt weniger als 70 mm und umfasst ein Vielzahl von analytischen Testmitteln (z.B. Stech- und/oder Analyseeinheiten) in zugeordneten Magazineinheiten beispielsweise in einer Gesamtzahl im Bereich von 10 bis 100 Einheiten. Vorteilhaft ist es auch, wenn die Transportelemente durch einen Kranz von an jeweils einer Magazineinheit achsparallel oder radial ausgerichteten Vorsprüngen (z.B. Zapfen) oder Vertiefungen gebildet sind.

In verfahrensmäßiger Hinsicht wird die eingangs genannte Aufgabe dadurch gelöst, dass ein Magazin als Verbrauchsmittel in einer Magazinführung eines handgehaltenen bzw. mobilen Geräts eingesetzt wird, wobei das Magazin eine Mehrzahl von mit jeweils mindestens einem analytischen Hilfsmittel und einem Transportelement versehene Magazineinheiten umfasst, dass das Magazin durch einen an den vorzugsweise ringförmig angeordneten Transportelementen angreifenden Transportmechanismus schrittweise in der Magazinführung transportiert wird, und dass Haltemittel einer geräteseitigen Positioniereinrichtung mit Transportelementen des Magazins in Eingriff gebracht werden, wobei eine zur Benutzung bereitgestellte aktive Magazineinheit in einer vorgegebenen Funktionsposition gerätefest positioniert wird.

Für eine möglichst genaue Positionierung am Messort ist es vorteilhaft, wenn über Haltemittel an einem Transportelement im Bereich der aktiven Magazineinheit angegriffen und eine Bewegung des Magazins in mindestens einer Richtung verhindert wird. Vorteilhaft ist es auch, wenn über eine Blockierung mindestens eines von der aktiven Magazineinheit (möglichst weit) entfernten Transportelements das Magazin während einer Positionierphase unbeweglich gehalten wird.

Bei der Positionierung wird erfindungsgemäß die niedrige Geometrie des Magazins mit ihrer vergleichsweise großen Grundfläche genutzt, dem Magazin eine Arbeitsebene zuzuweisen. Das geschieht vorteilhaft dadurch, dass die große Grundfläche des Magazins auf drei geräteseitigen Auflageflächen aufliegt und mit jeweils lotrecht dazu wirkenden Kräften dort gehalten wird. Damit werden bereits drei Bewegungsfreiheitsgrade festgelegt.

Zur weiteren Lagefixierung wird auf eine Lagerung zwischen Spitzen zurückgegriffen, um das Magazin entlang einer Achse definiert raumfest zu halten. Dabei werden zwei der Haltemittel mit geeigneten Transportelementen der Magazineinheiten in Kontakt gebracht, wobei das Haltemittel, das an der momentan aktiven Magazineinheit angreift, zwei weitere Freiheitsgrade fixiert, während das Haltemittel, das diametral bzw. möglichst weit entfernt dazu angreift, den letzten (sechsten) Freiheitsgrad festlegt. Denkbar ist es auch, dass zwischen zwei möglichst weit von der aktiven Magazineinheit entfernte Transportelemente eingegriffen wird, so dass ebenfalls eine statisch bestimmte Lagerung entsteht, z.B. indem ein Keilstück so zwischen zwei Transportelemente greift, dass diese auf den Keilflanken aufliegen.

Vorteilhaft wird das messortnahe Haltemittel durch eine starre Gerätestruktur und das messortferne Haltemittel in Form einer beweglichen Gerätestruktur verwirklicht. Letztere sollte mit einer Kraft entsprechend der maximal zulässigen Stoßbelastung beaufschlagt werden, um eine stoßsichere Positionierung zu gewährleisten.

Um das Magazin bei Bedarf aus dieser fixierten Position heraus in einer nachfolgenden Transportphase um eine Testeinheit weiter zu bewegen, werden erfindungsgemäß nacheinander genau die Freiheitsgrade gelöst, in denen das Magazin bewegt werden soll, wobei die an sich störenden Reibungen durch den Andruck des Magazins auf seine Arbeitsebene dazu genutzt werden, um das Magazin trotz der Freigabe von Freiheitsgraden daran zu hindern, unkontrolliert seine Position zu verlassen.

Vorteilhafterweise wird das ringförmige Magazin an dem Transportelement der aktiven Magazineinheit exzentrisch gelagert, so dass in der Transportphase eine begrenzte Schwenkbewegung des ganzen Magazins möglich ist.

Im Folgenden wird die Erfindung anhand eines in der Zeichnung dargestellten Ausführungsbeispiels näher erläutert. Es zeigen:
- Fig. 1: ein diagnostisches Analysesystem umfassend ein Handgerät und ein darin eingesetztes Magazin für analytische Hilfsmittel in einer perspektivischen Darstellung;
- Fig. 2: das in Form eines Rings bzw. einer Scheibe ausgebildete Magazin in einer perspektivischen Darstellung;
- Fig. 3: eine Magazineinheit in einem abgebrochenen Radialschnitt des Magazins;
- Fig. 4 - 6: aufeinanderfolgende Stellungen eines Transportmechanismus in Verbindung mit einer Positioniereinrichtung für das Magazin in Draufsicht.

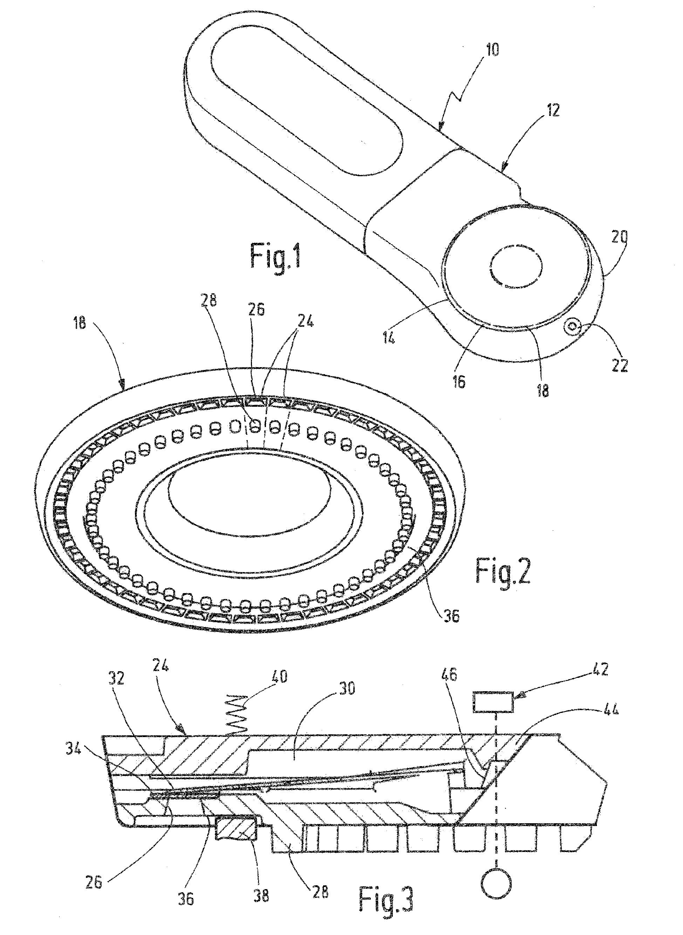

Das in Fig. 1 dargestellte Analysesystem 10 lässt sich von einem Probanden in der Hand halten und dabei für Blutzuckermessungen vor Ort einsetzen. Das Instrument umfasst zu diesem Zweck ein Mobil- bzw. Handgerät 12 mit einer mittels Deckel 14 verschließbaren Magazinführung 16 zur Aufnahme eines disposiblen Ringmagazins 18. Am Gehäuse 20 des Geräts 12 ist eine Applikationsöffnung 22 zum Ansetzen eines Fingers des Probanden vorgesehen, um eine Blutprobe für eine Glukosebestimmung durch einen Hauteinstich zu gewinnen.

Das in Fig. 2 gezeigte kreisringförmige Ringmagazin 18 weist eine Vielzahl von in Umfangsrichtung verteilten Magazineinheiten 24 auf. Jede Magazineinheit 24 besitzt ein Messfenster 26 und ein Transportelement 28 in Form eines an der Magazinunterseite abstehenden Zapfens. Die ringförmig angeordneten Transportelemente 28 ermöglichen ein schrittweises Weiterschalten und definiertes Positionieren der einzelnen Magazineinheiten 24 in einer Funktionsposition vor der Applikationsöffnung 22.

Wie in Fig. 3 gezeigt, befinden sich in der Magazinkammer 30 jeder Magazineinheit 24 ein Stechelement 32 und ein Testelement 34. Das Stechelement 32 lässt sich in der Funktionsposition mittels eines nicht gezeigten hin- und hergehenden Stechantriebs radial ausstoßen, um bei einem Hauteinstich eine geringe Blutmenge in einem Kapillarkanal aufzunehmen und auf das Testfeld 34 zu übertragen. Das Testfeld 34 ist mit einer Testchemie versehen, die auf den Analyten (Glukose) durch eine Farbänderung anspricht und über das Messfenster 26 mittels einer nicht gezeigten photometrischen Messeinheit quer zu der Ringebene optisch abgetastet werden kann. Dabei ist es besonders wichtig, die aktive Magazineinheit in der Funktionsposition vor der Applikationsöffnung 22 und über der Messeinheit so genau wie möglich zu positionieren. Zu diesem Zweck weist das Magazin 18 neben den Transportelementen 18 eine Planfläche 36 auf, die durch eine geräteseitige Dreipunktlagerung auf gehärteten Auflagehöckern 38 in einem definierten Messabstand gehalten wird, so dass der optische Strahlengang reproduzierbar ist. Um die Lagetoleranzen gering zu halten, drückt auf der Gegenseite jeder der drei trigonal-planar angeordneten Auflagehöcker 38 eine Feder 40 auf die Oberseite des Magazins 18.

Um insbesondere bei einem Magazinwechsel zu verhindern, dass benutzte Stechelemente wieder zum Einsatz kommen, ist eine geräteseitige Lichtschranke 42 vorgesehen, die einen Klarsichtbereich 44 der Magazineinheiten 24 durchstrahlt. Dabei wird eine die Magazinkammer 30 gegenüber dem Stechantrieb verschließende Steril- bzw. Siegelfolie 46 abgetastet, bei deren Zerstörung der Gebrauchszustand direkt erfassbar ist.

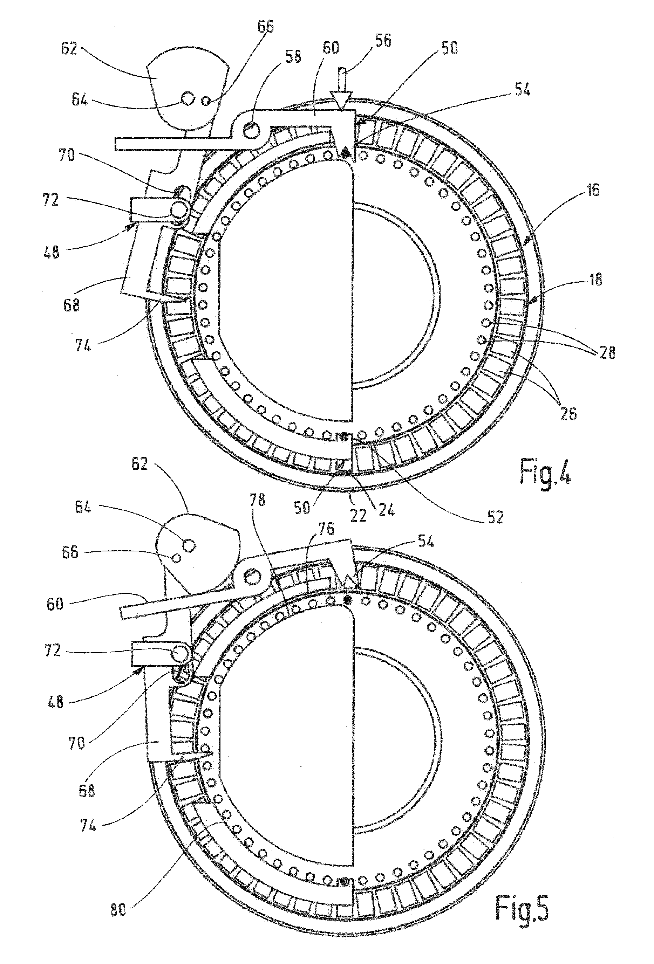

Die Fig. 4 bis 7 veranschaulichen den Bewegungsablauf bei der Weiterschaltung und definierten Positionierung der Magazineinheiten 24 des Magazins 18 in dem nicht eigens dargestellten Gerät 12. Zu diesem Zweck ist ein geräteseitiger Transportmechanismus 48 mit einer Positioniereinrichtung 50 des Geräts gekoppelt, um durch eine Manipulation der Transportelemente 28 des Magazins 18 die genannten Funktionen zu erfüllen.

Fig. 4 zeigt eine Magazinstellung, in der eine Magazineinheit 24 in der Funktionsposition lagefest gehalten ist. Hierfür sind geräteseitig angeordnete Haltemittel 52, 54 der Positioniereinrichtung 50 vorgesehen, die mit zwei einander diametral gegenüberliegenden Transportelementen 28 (in der Zeichnung schwarz hervorgehoben) des Magazins 18 in lösbarer Formschlussverbindung stehen.

Eines der Haltemittel ist durch ein seitlich offenes Lagerauge 52 gebildet, welches als gerätefeste Lagerstelle das Transportelement 28 der in der Funktionsposition befindlichen Magazineinheit 24 halbseitig umgreift. Aufgrund des geringen Abstandes zu dem aktiven Messfenster 26 dieser Magazineinheit 24 wird eine geringe Lagetoleranz erreicht. Dabei wird durch die steilen Flanken des Lagerauges eine Selbsthemmung gegenüber Querbewegungen bewirkt.

Somit könnte das Magazin 18 nur noch um den festgelegten Zapfen rotieren bzw. linear aus dem Lagerauge herausrücken. Um auch eine Bewegung in diesen verbleibenden Freiheitsgraden zu verhindern, greift ein prismatischer Greifer 54 als Haltemittel an dem diametral gegenüberliegenden Zapfen an. Dieser Greifer 54 wird durch eine Rückstellkraft beispielsweise mittels Feder in Richtung des Pfeils 56 gedrängt. Die Rückstellkraft sollte größer als die Reibungskräfte sein, die durch die Anfederung des Magazins 18 auf den drei Lagerhöckern 38 hervorgerufen werden. Um eine Kopplung mit dem Transportmechanismus 48 zu ermöglichen, ist der Greifer 54 an einem Ende eines um die Achse 58 schwenkbaren zweiarmigen Greiferhebels 60 angeordnet.

In dieser Anordnung befindet sich die aktive Magazineinheit 24 geometrisch eindeutig vor der Messeinheit in Funktionsposition. Der Greifer 54 als einzige bewegliche Struktur, die daran beteiligt ist (und z.B. für ihre Bewegung ein Spiel benötigt), ist mit einem Abstand entsprechend dem Durchmesser des Zapfenrings von der lagebestimmenden Lagerstelle 52 sehr viel weiter entfernt als die im Bereich des Messfensters 26 befindliche Messeinheit, die somit einen genau definierten optischen Übertragungsweg besitzt.

Durch die selbsthemmende Ausführung der Haltemittel 52, 54 für die Zapfen bzw. Transportelemente 28 ist ein Weitertransport des Ringmagazins 18 zur nächsten Magazineinheit 24 allerdings unterbunden. Um diesen Transport zu ermöglichen, muss der Greifer 54 mit seinem Fangprisma von dem Transportmechanismus 48 aktiv gegen die Rückstellkraft 56 aus seiner Eingriffstellung in eine Freigabestellung ausgehoben werden, wie es in den Fig. 5 bis 7 schrittweise dargestellt ist.

Zur Bewegungskopplung weist der Transportmechanismus 48 einen Steuernocken 62 als Kurvengetriebemittel auf, der auf der Abtriebswelle 64 eines Motors sitzt und über einen Kurbelzapfen 66 einen Baggerhebel 68 antreibt, während die Umfangskontur des Steuernockens 62 den Greiferhebel 60 betätigt. Der Baggerhebel 68 ist über ein Langloch 70 so gelagert, dass er in einer insgesamt elliptischen Bewegung an einem gerätefesten Lagerzapfen 72 vor und zurück laufen sowie sich um diesen drehen kann. Im Zuge dieser Bewegung greift ein klingenförmiger Schieber 74 an den Transportelementen 28 an und bewirkt im zusammenspiel mit bogenförmigen Leitkonturen 76, 78, 80 (Fig. 5) der Magazinführung 16 einen Transportschritt zur nächsten Magazineinheit 24.

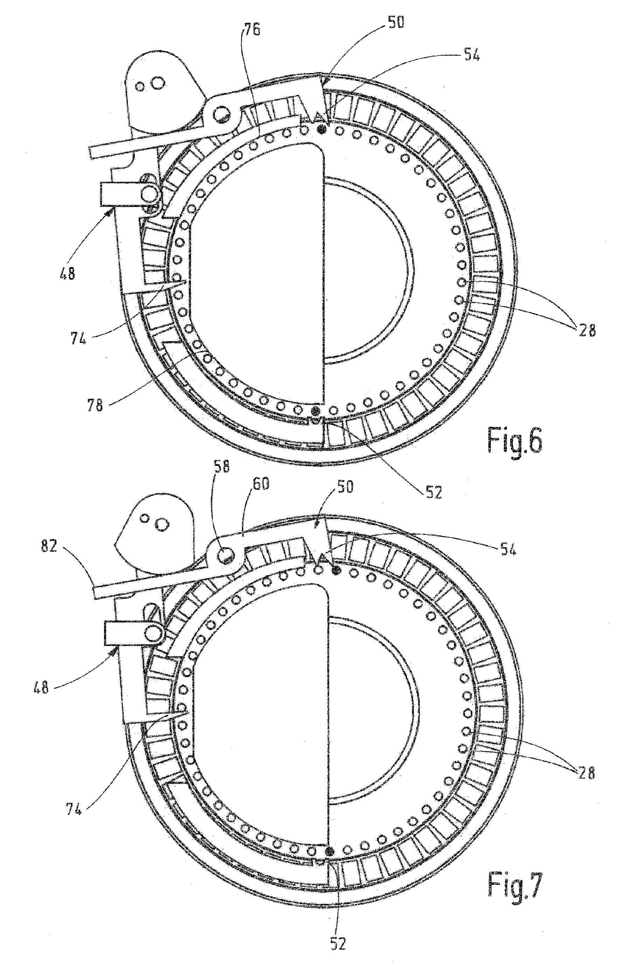

In der in Fig. 5 gezeigten Anfangsphase der Weiterschaltung wird zunächst der Greifer 54 durch den Steuernocken 62 in seine Freigabestellung bewegt. Simultan dazu wird der Schieber 74 auf einer elliptischen Bahn so in den Kranz von Transportelementen 28 geführt, dass eines davon erfasst wird. Bedingt durch den stark exzentrischen Angriff dieser Schieberbewegung relativ zur Reibung der Anpressfedern 40 gegen die drei Auflageflächen des Magazins 18 entsteht ein Drehmoment, das das ganze Magazin 18 um das in dem Lagerauge 52 befindliche Transportelement verschwenken lässt (im Uhrzeigersinn in Fig. 5). Diese Schwenkbewegung wird begrenzt durch die bogenförmige Leitkontur 76, gegen die der Kranz von Transportelementen 28 anläuft (Fig. 6). Sobald die Schwenkbewegung gestoppt ist, bleibt nur noch ein linearer Freiheitsgrad übrig, der es dem Magazin erlaubt, aus dem Lagerauge 52 auszurücken, bis wiederum Transportelemente 28 an der Leitkontur 78 anstoßen, so dass in der Endphase nur noch eine Rotation des ganzen Magazins 18 möglich ist (Fig. 7). Nachdem diese Rotation eine Magazinteilung weit erfolgt ist, kommt der Schieber 74 auf seinem elliptischen Weg außer Eingriff mit den Transportelementen 28 und der Greifer 54 senkt sich wieder auf das in seinem Fangbereich befindliche nächste Transportelement, wodurch das Magazin 18 auch wieder in das Lagerauge 52 einrückt. Das Magazin 18 ist somit wieder eindeutig vor der Messeinheit positioniert, aber mit einer neuen Magazineinheit 24.

Der vorstehend beschriebene Mechanismus gibt das Magazin 18 nie vollständig frei. Bei einer Magazinentnahme aus dem System würde der Greifer 54 unter der Rückstellkraft 56 noch weiter in die Magazinführung 16 einschwenken, wodurch das Einlegen eines neuen Magazins behindert würde. Um dies zu vermeiden, ist der Greiferhebel 60 verlängert und an seinem freien Ende 82 über ein nicht gezeigtes Stellmittel mit dem Gerätedeckel 14 gekoppelt. Das Stellmittel, z.B. eine Pleuelstange wird vom aufklappenden Gerätedeckel 14 verschoben und betätigt den Greiferhebel 60, so dass der Greifer 54 in seine Freigabestellung gelangt, und zwar ohne dabei den Schieber 74 in Eingriff zu nehmen.

Dies erlaubt es dem Anwender, ein neues Magazin in das Gerät zu legen, ohne auf die Winkelausrichtung des Magazins 18 zu achten. Um zu vermeiden, dass das Magazin 18 in einer Zwischenposition liegen bleibt und von dem wieder einschwenkenden Greifer 54 in dieser Zwischenposition festgehalten wird, sollte nach dem Schließen des Gehäusedeckels 14 zunächst ein Transportzyklus erfolgen. Außerdem sollte sichergestellt sein, dass beim Einwechseln eines bereits benutzten Magazins keine benutzten Stechelemente 32 zum Einsatz kommen, um eine Infektionsgefahr auszuschließen. Hierfür sollten die Transportzyklen so lange absolviert werden, bis mittels der Lichtschranke 42 als Prüfeinheit eine ungebrauchte Magazineinheit 24 in der Funktionsposition detektiert wird.

## Patentansprüche

1. Analytisches System zur Untersuchung einer Körperflüssigkeit, insbesondere für Blutzuckertests, mit
a) einem als Verbrauchsmittel wechselbaren Magazin (18), das eine Mehrzahl von mit jeweils mindestens einem analytischen Hilfsmittel (32,34) und einem Transportelement (28) versehene Magazineinheiten (24) umfasst,
b) einem handgehaltenen Gerät (12) mit einer Magazinführung (16) zur Aufnahme des Magazins (18),
c) einem an den vorzugsweise ringförmig angeordneten Transportelementen (28) angreifenden Transportmechanismus (48) zum schrittweisen Transport des Magazins (18) in der Magazinführung (16),
**gekennzeichnet durch**
d) eine über Haltemittel (52,54) mit Transportelementen (28) des Magazins (18) in Eingriff bringbare Positioniereinrichtung (50) zur Positionierung einer aktiven Magazineinheit (24) in einer vorgegebenen Funktionsposition.

2. Analytisches System nach Anspruch 1, **dadurch gekennzeichnet, dass** Haltemittel (52,54) an dem Transportelement (28) der aktiven Magazineinheit (24) formschlüssig angreifen und eine Bewegung des Magazins (18) in mindestens einer Richtung blockieren.

3. Analytisches System nach Anspruch 1 oder 2,
**dadurch gekennzeichnet, dass** die Haltemittel (52,54) eine in dem Gerät starr angeordnete Lagerstelle (52) zur Aufnahme des Transportelements (28) der aktiven Magazineinheit (24) umfassen.

4. Analytisches System nach Anspruch 3, **dadurch gekennzeichnet, dass** das ringförmige Magazin (18) in der Lagerstelle (52) um eine durch das Transportelement (28) der aktiven Magazineinheit verlaufende exzentrische Achse begrenzt schwenkbeweglich gelagert ist.

5. Analytisches System nach einem der Ansprüche 1 bis 4, **dadurch gekennzeichnet, dass** die Haltemittel (52,54) einen zwischen einer mit einem Transportelement (28) in Eingriff befindlichen Eingriffsstellung und einer davon entfernten Freigabestellung beweglichen Greifer (54) zur Lagesicherung des Magazins (18) umfassen.

6. Analytisches System nach Anspruch 5, **dadurch gekennzeichnet, dass** der Greifer (54) in seiner Eingriffsstellung unter Ausübung einer Kraft gegen mindestens ein Transportelement (28) andrückt.

7. Analytisches System nach Anspruch 5 oder 6,
**dadurch gekennzeichnet, dass** der Transportmechanismus (48) ein Steuermittel (62), insbesondere ein Kurvengetriebe zur Bewegungssteuerung des Greifers (54) aufweist.

8. Analytisches System nach einem der Ansprüche 5 bis 7, **dadurch gekennzeichnet, dass** der Greifer (54) über ein Stellmittel mit einem die Magazinführung (16) verschließenden Deckel (14) gekoppelt ist, so dass der Greifer (54) beim Öffnen des Deckels (14) in die Freigabestellung gelangt.

9. Analytisches System nach einem der Ansprüche 1 bis 8, **dadurch gekennzeichnet, dass** die Magazinführung (16) drei eine Ebene aufspannende Auflagepartien (38) zur Auflagerung einer ebenen Führungsfläche (36) des Magazins (18) aufweist.

10. Analytisches System nach einem der Ansprüche 1 bis 9, **dadurch gekennzeichnet, dass** das in einer Lagerebene begrenzt bewegbare Magazin (18) unter der Einwirkung einer Normalkraft zu der Lagerebene steht.

11. Analytisches System nach einem der Ansprüche 1 bis 10, **dadurch gekennzeichnet, dass** der Transportmechanismus (48) mit Getriebemitteln (66,68) versehen ist, die einer exzentrischen Schwenkbewegung des Magazins (18) eine lineare Ausrückbewegung überlagern, wobei mindestens ein Transportelement (28) außer Eingriff mit einem gerätefesten Haltemittel (52,54) gelangt.

12. Analytisches System nach einem der Ansprüche 1 bis 11, **dadurch gekennzeichnet, dass** der Transportmechanismus (48) einen auf einer elliptischen Bahn bewegbaren und in einem Abschnitt seiner Bewegungsbahn an einem Transportelement (28) angreifenden Schieber (74) aufweist.

13. Analytisches System nach einem der Ansprüche 1 bis 12, **dadurch gekennzeichnet, dass** die Magazinführung (16) bogenförmige Leitkonturen (76,78,80) für die Transportelemente (28) aufweist, wobei die Leitkonturen (76,78,80) eine von einer Kreisbahn abweichende Ausrückbewegung der Transportelemente (28) steuern.

14. Analytisches System nach einem der Ansprüche 1 bis 13, **gekennzeichnet durch** eine Prüfeinheit (42) insbesondere in Form einer eine Siegelfolie (46) abtastenden Lichtschranke zur Gebrauchsprüfung der Magazineinheiten (24).

15. Verfahren zum Betrieb eines analytischen Systems insbesondere nach einem der vorhergehenden Ansprüche, bei dem
- ein Magazin (18) als Verbrauchsmittel in einer Magazinführung (16) eines handgehaltenen Geräts (12) eingesetzt wird, wobei das Magazin (18) eine Mehrzahl von mit jeweils mindestens einem analytischen Hilfsmittel (32,34) und einem Transportelement (28) versehene Magazineinheiten (24) umfasst,
- das Magazin (18) durch einen an den vorzugsweise ringförmig angeordneten Transportelementen (28) angreifenden Transportmechanismus (48) schrittweise in der Magazinführung (16) transportiert wird,
**dadurch gekennzeichnet, dass**
- dass Haltemittel (52,54) einer geräteseitigen Positioniereinrichtung (50) mit Transportelementen (28) des Magazins (18) in Eingriff gebracht werden, wobei eine zur Benutzung bereitgestellte aktive Magazineinheit (24) in einer vorgegebenen Funktionsposition gerätefest positioniert wird.
